# EUROPÄISCHE PATENTANMELDUNG

(11) **EP 4 793 859 A1**
(43) Veröffentlichungstag der Anmeldung: **19.08.2026**
(21) Anmeldenummer: 26151505.0
(22) Anmeldetag: 13.01.2026
(51) Int. Cl.: G06Q 30/015, G06Q 30/0282, G06Q 30/0251, G06Q 30/0601, G06Q 10/40, A47J 36/32

(54) **VERFAHREN UND SYSTEM ZUM AUTOMATISCHEN ANLEGEN EINES NUTZERPROFILS**

(30) Priorität: 12.02.2025 BE 202500014
(71) Anmelder: Miele & Cie. KG, 33332 Gütersloh (DE)
(72) Erfinder: Carsten, Daniel, 33615 Bielefeld (DE); Frese, Maksim, 33102 Paderborn (DE); Köpp, Lukas, 33378 Rheda-Wiedenbrück (DE)

(57) **Zusammenfassung**

Die Erfindung betrifft ein Verfahren und ein System (100) zum automatischen Anlegen eines Nutzerprofils (102) zur Nutzung eines Küchengerätes (104) und zum Bereitstellen einer Assistenzfunktion (106), das Verfahren aufweisend die Schritte: Bereitstellen von Nutzungsinformationen (109) zur Nutzung des Küchengerätes (104) und/oder zu einem Nutzer des Küchengerätes (104) und/oder Lebensmitteln, die mit dem Küchengerät (104) zubereitet werden; Anlegen des Nutzerprofils durch einen Softwaremodul-Agenten unter Verwendung der bereitgestellten Nutzerinformationen; und Bereitstellen der Assistenzfunktion (106) unter Verwendung des angelegten Nutzerprofils (102).

## Beschreibung

Die Erfindung betrifft ein Verfahren und ein System zum automatischen Anlegen eines Nutzerprofils.

Im Stand der Technik existieren verschiedene Ansätze, die sich mit der Erfassung und Verwaltung von Informationen zu Ernährungsgewohnheiten sowie mit der Nutzung von Rezepten und Automatikprogrammen für Küchengeräte befassen. So gibt es beispielsweise Anwendungen, die das Führen von Essenstagebüchern oder Einkaufslisten ermöglichen. Nutzer können dort Mahlzeiten, Rezepte oder benötigte Zutaten manuell eintragen. Für den Einkauf und die Bestellung von Lebensmitteln stehen ebenfalls Apps zur Verfügung, die es erlauben, Zutaten auszuwählen oder abzuwählen und Hinweise oder besondere Wünsche an Lieferdienste zu übermitteln. In Restaurants besteht zudem die Möglichkeit, spezifische Lebensmittelvorlieben oder Unverträglichkeiten direkt der Bedienung mitzuteilen, um entsprechende Anpassungen der Gerichte vornehmen zu lassen.

Auf der Seite der Küchengerätehersteller ist die Entwicklung von Automatikprogrammen ein zeit- und ressourcenintensiver Prozess. Um solche Programme bereitzustellen, müssen Hersteller Rezepte entwickeln, diese mit den eigenen Geräten testen und die erforderlichen Parameter validieren, bevor die Programme den Kunden zugänglich gemacht werden können. Zwar gibt es bereits lieferantenseitig vorgegebene Automatikprogramme, die teilweise auch per Update auf Hausgeräte geladen werden können, jedoch fehlen bisher Lösungen, die eine automatische Erstellung individueller Nutzerprofile ermöglichen. Ebenso gibt es keine bekannten Anwendungen, die das Teilen solcher Nutzerprofile mit anderen gestatten, um Vorlieben, Abneigungen, Unverträglichkeiten oder Allergien in einer gemeinschaftlichen Weise zu berücksichtigen.

Die derzeitigen Lösungen erfordern vom Nutzer in der Regel aktive Eingaben und eine manuelle Pflege der Daten, was insbesondere bei Essenstagebüchern und Nutzerprofilen aufwendig und unpraktisch sein kann. Automatisierte Systeme, die Daten aus einer vernetzten Küche sammeln, diese in ein Nutzerprofil integrieren und für verschiedene Zwecke wie die Filterung großer Rezeptsammlungen, die Bereitstellung personalisierter Gesundheitshinweise oder die Validierung neuer Rezepte nutzen, sind bisher nicht verfügbar.

Es ist somit eine Aufgabe, ein verbessertes Verfahren und/oder ein verbessertes System anzugeben.

Erfindungsgemäß wird die Aufgabe durch ein Verfahren mit den Merkmalen des Patentanspruchs 1 gelöst. Ferner wird die Aufgabe durch ein System mit den Merkmalen des Patentanspruchs 14 gelöst. Vorteilhafte Ausgestaltungen und Weiterbildungen der Erfindung ergeben sich aus den nachfolgenden Unteransprüchen.

Demgemäß wird ein Verfahren zum automatischen Anlegen eines Nutzerprofils zur Nutzung eines Küchengerätes und zum Bereitstellen einer Assistenzfunktion vorgeschlagen. Das Verfahren weist die Schritte auf: Bereitstellen von Nutzungsinformationen zur Nutzung des Küchengerätes und/oder zu einem Nutzer des Küchengerätes und/oder Lebensmitteln, die mit dem Küchengerät zubereitet werden; Anlegen des Nutzerprofils durch einen Softwaremodul-Agenten unter Verwendung der bereitgestellten Nutzerinformationen; und Bereitstellen der Assistenzfunktion unter Verwendung des angelegten Nutzerprofils.

Ferner wird ein System zum automatischen Anlegen eines Nutzerprofils zur Nutzung eines Küchengerätes und zum Bereitstellen einer Assistenzfunktion vorgeschlagen. Das System umfasst das Küchengerät, ein Rechenmodul zur Ausführung eines Softwaremodul-Agenten und ein Bereitstellungsmodul. Das Bereitstellungsmodul ist dazu ausgebildet, Nutzungsinformationen zur Nutzung des Küchengerätes und/oder zu einem Nutzer des Küchengerätes und/oder Lebensmitteln, die mit dem Küchengerät zubereitet werden, bereitzustellen. Das Rechenmodul ist dazu ausgebildet, das Nutzerprofil mittels des Softwaremodul-Agenten unter Verwendung der bereitgestellten Nutzerinformationen anzulegen. Das Bereitstellungsmodul ist ferner dazu ausgebildet, die Assistenzfunktion unter Verwendung des angelegten Nutzerprofils bereitzustellen.

Ein Nutzerprofil zur Nutzung eines Küchengerätes umfasst vorzugsweise personenbezogene und/oder nutzerspezifische Daten, die zur individuellen Anpassung und Optimierung der Verwendung des Küchengerätes erfasst und/oder gespeichert werden. Ein Nutzerprofil zur Nutzung eines Küchengerätes kann vorzugsweise Informationen über Ernährungsgewohnheiten, Vorlieben, Abneigungen, Unverträglichkeiten und/oder Allergien eines Nutzers enthalten. Zudem kann ein Nutzerprofil zur Nutzung eines Küchengerätes Informationen über häufig verwendete Rezepte, bevorzugte Zubereitungsarten und/oder Geräteeinstellungen umfassen. Ein Nutzerprofil zur Nutzung eines Küchengerätes kann weiterhin genutzt werden, um Empfehlungen zu geben, neue Rezepte zu filtern oder Automatikprogramme zu personalisieren.

Nutzungsinformationen zur Nutzung des Küchengerätes und/oder zu einem Nutzer des Küchengerätes und/oder Lebensmitteln umfassen vorzugsweise Daten, die sich auf die Interaktion des Nutzers mit dem Küchengerät und/oder auf die verwendeten Lebensmittel beziehen. Nutzungsinformationen zur Nutzung des Küchengerätes und/oder zu einem Nutzer des Küchengerätes und/oder Lebensmitteln können beispielsweise die Häufigkeit und Art der Nutzung des Gerätes umfassen. Ebenso können Nutzungsinformationen zur Nutzung des Küchengerätes und/oder zu einem Nutzer des Küchengerätes und/oder Lebensmitteln Angaben über bevorzugte oder vermiedene Lebensmittel enthalten. Ferner können Nutzungsinformationen zur Nutzung des Küchengerätes und/oder zu einem Nutzer des Küchengerätes und/oder Lebensmitteln dazu verwendet werden, um personalisierte Empfehlungen zu erstellen oder die Funktionalität des Gerätes an die individuellen Bedürfnisse des Nutzers anzupassen. Nutzungsinformationen zur Nutzung des Küchengerätes und/oder zu einem Nutzer des Küchengerätes und/oder Lebensmitteln können außerdem eine Grundlage für das automatische Erstellen von Nutzerprofilen sein.

Insbesondere hat bislang eine Möglichkeit gefehlt, automatisch generierte Profile mit anderen zu teilen oder Rezeptänderungen und individuelle Anpassungen in einer Koch-Community zu speichern und zu validieren. Dies vereinfacht jedoch die Entwicklung neuer, validierter Rezepte für Küchengeräte erheblich und bereichert die Nutzung solcher Geräte durch ein erweitertes Angebot an Rezepten und Programmen.

Das Anlegen der Nutzerprofile verschiedener Nutzer bietet den Vorteil, dass das Planen und/oder Zubereiten für bestimmte Personen und/oder für eine größere Gruppe von Personen einfacher wird. Das Absprechen und Berücksichtigen von Lebensmittelvorlieben, bzw. -abneigungen und/oder Allergien, das sich sonst als herausfordernd erweisen kann, wird durch das vorliegende Verfahren erheblich vereinfacht. Ernährungsbewusste Nutzer profitieren ferner davon, dass durch die Bereitstellung ihres Nutzerprofils an Restaurants oder Lieferdiensten auf ihre nutzerspezifischen Ernährungsbedürfnisse eingegangen werden kann. Ferner können Haushaltsgerätehersteller von solchen Nutzerprofilen profitieren, da beim Teilen und/oder Validieren von persönlichen oder externen Daten, wie Rezepten, schnell und einfach neue Automatikprogramme für viel verwendete Rezepte erlernt und den weiteren Nutzern zur Verfügung gestellt werden können. Dadurch können Kosten für die Erstellung solcher Automatikprogramme eingespart werden.

Durch das vorliegende Verfahren kann beispielsweise auch im Alltag des Nutzers automatisch ein Nutzerprofil über dessen Vorlieben und Abneigungen bzw. Unverträglichkeiten von Lebensmitteln erstellt werden, ohne dass der Nutzer sich hierzu Zeit nehmen muss. Die Erstellung des Nutzerprofils erfolgt vielmehr automatisch und im Hintergrund ohne die offensichtliche Wahrnehmung des Nutzers. Das Nutzerprofil kann vorzugsweise auch dynamisch und fortlaufend erweitert werden, um derart auch Änderungen und/oder Anpassungen über die Zeit, beispielsweise veränderte Ernährungsgewohnheiten, automatisch zu erkennen. Das so angelegte Nutzerprofil kann die Basis für ein Assistenzsystem bilden, durch das dann beispielsweise Rezeptvorschläge ausgegeben werden können und/oder eine Ernährungsberatung erfolgen kann. Ferner kann das Nutzerprofil mit anderen Nutzern geteilt werden, um so beispielsweise bei der Erstellung von Menüs und/oder der Zubereitung von Gerichten auf diese Nutzerprofile Rücksicht nehmen zu können. Zudem können über ein Community-System mittels der Nutzerprofile schnell und einfach neue Automatikprogramme für das Küchengerät erstellt werden.

**In** einem vorliegenden System kann beispielsweise ein personalisiertes Nutzerprofil angelegt werden. **In** diesem Nutzerprofil werden vorzugsweise automatisch Informationen gespeichert. Zu diesen Informationen gehören vorzugsweise gekochte Mahlzeiten, Rezepte, Automatikprogramme und/oder vom Nutzer darüber hinaus bereitgestellte Informationen. Ferner kann das Verfahren und/oder System um ein Lebensmittel-Bestandsmanagement ergänzt sein, durch das beispielsweise vorhandene bzw. eingelagerte Lebensmittel gespeichert sind. Ferner können die Nutzerinformationen auch um Bilddaten von Lebensmitteln ergänzt werden. Derartige Bilddaten können beispielsweise aus einem Kühlschrank und/oder Aufbewahrungsraum für Lebensmittel erfasst werden. Ferner ist es möglich, Informationen über Lebensmitteleinkäufe als weitere Nutzerinformationen bereitzustellen.

Aus all den gesammelten Informationen kann somit ein Nutzerprofil abgeleitet werden. Aus Rezepten können beispielsweise häufig verwendete Lebensmittel als Vorlieben des Nutzers gespeichert werden. Nicht verwendete Lebensmittel werden vorzugsweise als "nicht gegessene Nahrungsmittel" (beispielsweise per Default) gespeichert. Der Nutzer kann auch aktiv aufgefordert werden, Lebensmittelvorlieben und Abneigungen anzugeben, um zu ermitteln, ob es sich bei einigen Lebensmitteln um zufällig nie verwendete Nahrungsmittel, oder um Abneigungen, Unverträglichkeiten und/oder Allergien handelt. Derartige Informationen können von dem Nutzer selbstverständlich auch manuell über eine Nutzeroberfläche eingegeben werden, um derart die Erstellung des Nutzerprofils zu Beginn bereits zu optimieren.

In einem weiteren Aspekt wird vorgeschlagen, dass die bereitgestellte Assistenzfunktion unter Verwendung des angelegten Nutzerprofils dem Nutzer bei einer Auswahl von Lebensmitteln für die Zubereitung eines Gerichtes und/oder bei einem Einkauf von Lebensmitteln und/oder bei einer Auswahl eines mit dem Küchengerät zuzubereitenden Rezeptes und/oder bei einer Anpassung eines Rezeptes und/oder bei der Durchführung einer Lebensmittelzubereitung und/oder bei der Einhaltung diätischer Bedürfnisse und/oder bei einer Ernährungsberatung assistiert.

Die Assistenzfunktion kann dem Nutzer bei einer Auswahl von Lebensmitteln für die Zubereitung eines Gerichtes Empfehlungen basierend auf individuellen Vorlieben, Unverträglichkeiten oder vorrätigen Zutaten geben. Die Assistenzfunktion kann den Nutzer bei einem Einkauf von Lebensmitteln unterstützen, indem Einkaufslisten generiert oder alternative Produkte vorgeschlagen werden. Die Assistenzfunktion kann dem Nutzer bei einer Auswahl eines mit dem Küchengerät zuzubereitenden Rezepts helfen, indem sie Rezepte nach Geschmack, Ernährungszielen oder verfügbaren Zutaten filtert. Die Assistenzfunktion kann den Nutzer bei einer Anpassung eines Rezepts begleiten, indem Vorschläge zur Modifikation hinsichtlich Portionsgröße, Kaloriengehalt oder alternativer Zutaten gemacht werden. Die Assistenzfunktion kann den Nutzer bei der Durchführung einer Lebensmittelzubereitung unterstützen, indem sie Schritt-für-Schritt-Anleitungen, Automatisierungsoptionen oder Anpassungen der Geräteeinstellungen bereitstellt. Die Assistenzfunktion kann den Nutzer bei der Einhaltung diätischer Bedürfnisse durch Analyse und Bewertung von Rezepten in Bezug auf gesundheitliche Anforderungen und persönliche Ernährungsziele unterstützen. Die Assistenzfunktion kann den Nutzer bei einer Ernährungsberatung begleiten, indem sie auf Basis gesammelter Nutzungsinformationen individuelle Empfehlungen für eine ausgewogene Ernährung gibt.

Aus dem angelegten Nutzerprofil können beispielsweise auf den Nutzer angepasste Rezeptempfehlungen an den Nutzer vorgenommen werden. Beispielsweise kann durch die Verwendung eines Ampelsystems dem Nutzer anzeigen, ob ein Rezept ausschließlich Lebensmittel bzw. Nahrungsmittel enthält, die bereits von dem Nutzer konsumiert wurden (gekennzeichnet beispielsweise mit "grün") oder ob auch Lebensmittel bzw. Nahrungsmittel in dem Rezept umfasst sind, die von dem Nutzer bislang bisher nicht konsumiert wurden, und es sich daher potenziell um eine Abneigung, eine Unverträglichkeit und/oder um eine Allergie des Nutzers handeln könnte.

**In** einem weiteren Aspekt wird vorgeschlagen, dass die bereitgestellte Assistenzfunktion unter Verwendung des angelegten Nutzerprofils zumindest Teile der Nutzungsinformationen und/oder des Nutzerprofils über eine öffentliche Plattform mit anderen Nutzern teilt und/oder für andere Nutzer einsehbar macht, um dem Nutzer und/oder den anderen Nutzern der öffentlichen Plattform bei der Auswahl von Lebensmitteln und/oder Rezepten zu assistieren und/oder über die öffentliche Plattform bereitgestellte Rezepte auf Basis des angelegten Nutzerprofils des Nutzers und/oder auf Basis von Nutzerprofilen der anderen Nutzer anzupassen.

Die bereitgestellte Assistenzfunktion teilt vorzugsweise zumindest Teile der Nutzungsinformationen und/oder des Nutzerprofils über eine öffentliche Plattform mit anderen Nutzern, um den Austausch und die Vernetzung zwischen Nutzern zu ermöglichen. Die bereitgestellte Assistenzfunktion macht vorzugsweise für andere Nutzer relevante Informationen einsehbar, um personalisierte Empfehlungen, gemeinschaftliches Lernen oder die Anpassung von Rezepten zu erleichtern. Die bereitgestellte Assistenzfunktion ermöglicht vorzugsweise eine gezielte Auswahl der zu teilenden Informationen, um Datenschutz und individuelle Präferenzen der Nutzer zu berücksichtigen. Die bereitgestellte Assistenzfunktion kann vorzugsweise durch Freigabeoptionen gesteuert werden, sodass der Nutzer selbst bestimmt, welche Inhalte für andere Nutzer sichtbar sind. Die bereitgestellte Assistenzfunktion unterstützt vorzugsweise die kollaborative Entwicklung neuer Rezepte oder Küchengeräteanwendungen, indem sie den Austausch und die Validierung von Nutzererfahrungen ermöglicht. Die bereitgestellte Assistenzfunktion nutzt vorzugsweise die geteilten Informationen, um die Qualität und Vielfalt der Rezeptvorschläge oder Assistenzfunktionen kontinuierlich zu verbessern.

Das Nutzerprofil kann also vorzugsweise verwendet werden, um es im Rahmen einer Community-Plattform mit anderen Nutzern zu teilen. Werden beispielsweise Freunde zum Essen eingeladen, können die geteilten Nutzerprofile dazu verwendet werden, um einen Abgleich mit Rezepten zu machen, die gekocht werden sollen. Die Nutzerprofile müssen nicht vollständig angezeigt werden, sondern können rein beispielhaft im Rahmen eines Ampelsystems dem Gastgeber anzeigen, ob ein Rezept geeignet ist für die Gäste zu kochen, oder ob es Probleme bei gewissen Lebensmitteln geben könnte. Dabei muss auch nicht vollständig preisgegeben werden, bei welchem Gast oder bei welchem Lebensmittel die Probleme auftauchen, um Anonymität zu gewährleisten. Der Gastgeber kann so einfach ein Rezept auswählen, welches für alle Nutzerprofile zu passen scheint. Ein solches Rezept kann auch aus einer Datenbank durch Vergleichen der Nutzerprofile automatisch vorgeschlagen werden. Dies spart dem Nutzer Zeit.

Das gleiche Prinzip kann auch für Restaurants und Lieferdienste verwendet werden, um vorzugsweise auf Basis des Nutzerprofils abzugleichen, ob bei der Verwendung bestimmter Lebensmittel ggf. Probleme auftreten könnten. Nicht immer sind diese Informationen für den Gast, bzw. Besteller ausreichend transparent oder ein Menü wird ggf. auch falsch ausgewählt. Restaurants und Lieferdienste können so ein Menü auf einen Kunden individuell anpassen. Dies hat insbesondere im Bereich der Gourmet-Küche große Vorteile.

In einem weiteren Aspekt wird vorgeschlagen, dass ferner ein Erkennen von Rezepten in einer Mehrzahl von Nutzerprofilen, die durch eine vorbestimmte Anzahl von Nutzern der öffentlichen Plattform durchgeführt werden, erfolgt.

Dabei können beispielsweise Rezepte erkannt werden, die von mehreren Nutzern beispielsweise mehrmals zubereitet wurden, und sich somit gegebenenfalls einer großen Aufmerksamkeit erfreuen. Dabei können beispielsweise auch temporäre Nahrungsmitteltrends oder auch allgemeine Vorlieben ausschlaggebend sein. Das Erkennen solcher Rezepte erfolgt vorzugsweise durch Vergleichsalgorithmen, die die Mehrzahl von Nutzerprofilen auf solche Rezepte hin untersuchen. Dabei können Ähnlichkeitsvergleiche aus dem Natural Language Processing (NLP) Bereich, analytische Vergleichsverfahren und auch maschinelle Lernverfahren, wie große Sprachmodelle, eingesetzt werden, um solche Vergleiche durchzuführen.

In einem weiteren Aspekt wird vorgeschlagen, dass die erkannten Rezepte durch ein, insbesondere generatives, maschinelles Lernmodell analysiert werden, um Parameter und/oder Informationen aus den Rezepten zu extrahieren.

Dabei werden vorzugsweise Informationen über gekochte Rezepte des Nutzers gesammelt. Dies können Rezepte aus diversen online Quellen, wie z.B. von Chefkoch.de oder anderen Rezept-Websites, oder aus offline Quellen, wie Kochbüchern und/oder Magazinen, sein. Der Nutzer kann Offline-Rezepte beispielsweise auch digitalisieren, beispielsweise über eine Bildaufnahme, bereitstellen. Online-Rezepte kann der Nutzer durch das Angeben oder Anklicken eines Links bereitstellen. Ein generatives, maschinelles Lernmodell, beispielsweise ein Transformer-Modell, wird dann vorzugsweise dazu verwendet, das betreffende Rezept zu analysieren und basierend darauf dem Rezept wichtige Informationen, beispielsweise über Parameter, Lebensmittel und/oder Zubereitungsverfahren usw., zu entnehmen. Ferner kann über Web-Crawling auch direkt auf Online-Quellen zugegriffen werden. Die Plattform Chefkoch.de ist beispielsweise bekannt und umfasst viele Favoritenrezepte, die von vielen Nutzern hoch bewertet worden sind. So gibt es für bestimmte Basisgerichte, z.B. eine Lasagne, ein von Nutzern häufig verwendetes Rezept, welches immer wieder gekocht wird. Derartige Rezepte können vorliegend automatisch analysiert werden.

In einem weiteren Aspekt wird vorgeschlagen, dass durch den Softwaremodul-Agenten aus den erkannten oder analysierten Rezepten ein Automatikprogramm für das Küchengerät erstellt wird, das durch das Küchengerät ausführbar ist.

Der Softwaremodul-Agent erkennt oder analysiert vorzugsweise Rezepte und generiert daraus ein Automatikprogramm, das speziell für die Ausführung durch das Küchengerät konzipiert ist. Der Softwaremodul-Agent erstellt vorzugsweise das Automatikprogramm auf Basis der Rezeptinhalte, der erforderlichen Zubereitungsschritte und der spezifischen Geräteeinstellungen. Der Softwaremodul-Agent optimiert vorzugsweise das Automatikprogramm, indem er Parameter wie Temperatur, Garzeit oder Rührgeschwindigkeit an die Eigenschaften des jeweiligen Küchengerätes anpasst. Der Softwaremodul-Agent ermöglicht vorzugsweise eine automatische Umwandlung von Rezepten in ausführbare Programme, wodurch die manuelle Anpassung durch den Nutzer oder den Hersteller reduziert wird. Der Softwaremodul-Agent berücksichtigt vorzugsweise Nutzungsinformationen und gespeicherte Präferenzen, um das Automatikprogramm individuell auf den jeweiligen Nutzer abzustimmen. Der Softwaremodul-Agent kann vorzugsweise kontinuierlich aktualisierte oder von einer Community validierte Rezepte in Automatikprogramme umwandeln, sodass neue Programme effizient bereitgestellt werden können.

Es wird also beispielsweise immer wieder (bei mehreren Nutzern) das gleiche externe Rezept erkannt und analysiert. Dabei werden vorliegend die von den Kunden bei der Rezeptausführung verwendeten Parameter analysiert und ggf. auch automatisch an ein jeweiliges Küchengerät angepasst. Immer wieder verwendete und/oder angepasste Parameter werden dann vorzugsweise (beispielsweise in einer Datenbank) gespeichert. Wird ein Rezept einer externen (online) Quelle wiedererkannt, das von dem Nutzer zubereitet wird, kann vorliegend ein Abgleich mit für dieses Rezept bereits verwendeten Parametern von anderen vernetzten Nutzern erfolgen. Darauf basierend können Empfehlungen ausgegeben werden, wie z.B. "Andere Nutzer, die dieses Rezept gekocht haben, haben durchschnittlich eine um 10° höhere Temperatur eingestellt".

In einem weiteren Aspekt wird vorgeschlagen, dass dem Nutzer auf Basis von Informationen der öffentlichen Plattform, die zumindest teilweise den Teilen der Nutzungsinformationen und/oder des Nutzerprofils entsprechen, ein Anpassungsvorschlag zur Anpassung einer Geräteeinstellung des Küchengerätes ausgegeben oder die Anpassung der Geräteeinstellung automatisch an dem Küchengerät vorgenommen wird.

Dem Nutzer wird vorzugsweise ein Anpassungsvorschlag zur Anpassung einer Geräteeinstellung des Küchengerätes ausgegeben, basierend auf Informationen der öffentlichen Plattform, die zumindest teilweise den Teilen der Nutzungsinformationen und/oder des Nutzerprofils entsprechen. Dem Nutzer werden vorzugsweise Empfehlungen zur Optimierung der Geräteeinstellungen vorgeschlagen, um eine personalisierte Zubereitung entsprechend individueller Vorlieben oder gesammelter Erfahrungen anderer Nutzer zu ermöglichen. Dem Nutzer kann vorzugsweise eine automatische Anpassung der Geräteeinstellungen an dem Küchengerät vorgenommen werden, sodass die optimale Zubereitung ohne manuelle Eingriffe sichergestellt wird. Dem Nutzer stehen vorzugsweise konfigurierbare Optionen zur Verfügung, um zu bestimmen, ob Anpassungsvorschläge manuell bestätigt oder direkt umgesetzt werden sollen. Dem Nutzer wird vorzugsweise ermöglicht, von gemeinschaftlich gesammelten Erkenntnissen zu profitieren, indem er angepasste Geräteeinstellungen für bestimmte Rezepte oder Zubereitungsarten erhält. Dem Nutzer wird vorzugsweise ermöglicht, eigene Anpassungen zu speichern und mit der Plattform zu teilen, sodass zukünftige Anpassungsvorschläge weiter verbessert werden können.

In einem weiteren Aspekt wird vorgeschlagen, dass der Nutzer durch eine Nutzerrückmeldung über die öffentliche Plattform eine Bewertung zu einem Automatikprogramm und/oder zu einer Einstellung des Automatikprogramms des Küchengerätes abgibt, auf deren Basis eine Anpassung des Automatikprogramms erfolgt.

Ferner können beispielsweise auch Nutzerfeedbacks analysiert werden, um zu erkennen, ob die verwendeten Parameter zu einem gelungenen Ergebnis geführt haben. So entwickelt sich, insbesondere Schritt für Schritt, ein neues und validiertes Automatikprogramm für ein neues Rezept. Ein anderes Beispiel dafür ist ein, der allgemeinen Community zur Verfügung gestelltes, privates Rezept, mit der Beigabe von verwendeten Geräteparametern für das Küchengerät. Diese können auch automatisch durch das vernetzte Küchengerät an die Community-Plattform weitergegeben werden. Andere Küchengeräte-Nutzer kochen dieses private Rezept nun beispielsweise nach und verwenden dazu dieselben Parameter, oder lassen diese automatisch von dem vernetzten Küchengerät herunterladen und ausführen. Sollten die Ergebnisse gelungen sein, gibt der Nutzer vorzugsweise ein positives Feedback zu dem Rezept auf der Community-Plattform. Das System kann dieses Feedback dann vorzugsweise auswerten und damit die verwendeten Parameter zu einem solchen, privaten Rezept validieren. Derart kann sich ebenfalls ein Automatikprogramm für das Küchengerät entwickeln. Das Küchengerät kann dann diese Parameter dem Nutzer, der dieses Rezept nachkochen will, vorzugsweise vorschlagen. Dadurch können Nutzer vorzugsweise innerhalb der Community zu Experten werden und im Rahmen eines Stufensystems (User-Scoring) aufsteigen. Eingegebene, private Rezepte, selbst eingegebene Feedbacks, und/oder auf Rezepte eingegangene Feedbacks werden dafür ausgewertet und berücksichtigt und können so die Glaubwürdigkeit und die Aufnahme neuer Automatikprogramme regulieren.

In einem weiteren Aspekt wird vorgeschlagen, dass die bereitgestellte Assistenzfunktion unter Verwendung des angelegten Nutzerprofils zur Kommunikation mit einer mobilen Applikation ausgebildet ist, um dem Nutzer über die mobile Applikation mit Hinweisen zu assistieren.

Auf Basis des Nutzerprofils und auf Wunsch des Nutzers kann durch das vorliegende Verfahren auch eine Ernährungsberatung vorgenommen und/oder eine Hilfestellung bei der Nahrungsmittelauswahl gegeben werden. Dies kann vorzugsweise von dem Nutzer frei einstellbar sein. Dabei können beispielsweise Rezeptvorschläge und/oder Ernährungsratschläge gegeben werden, die beispielsweise Informationen umfassen, welche Nahrungsmittel öfter oder weniger oft verwendet werden sollten. Es können im Alltag auch Push-Nachrichten gesendet werden, die auf eine gezielte Nahrungseinnahme hinweist.

In einem weiteren Aspekt wird vorgeschlagen, dass die Nutzungsinformationen Informationen zu Lebensmitteln und/oder zu zubereiteten Lebensmitteln und/oder zu einem Rezept und/oder zu einem Rezeptergebnis und/oder zu einem an dem Küchengerät ausgewählten Automatikprogramm zum Zubereiten der Lebensmittel und/oder zu einer Geräteeinstellung des Küchengerätes und/oder zu Bild- und/oder Textdaten von den Lebensmitteln und/oder dem Rezept und/oder zu gelagerten Lebensmitteln und/oder zu Lebensmitteleinkäufen und/oder zu manuellen Nutzerinformationen aufweist.

Ferner können auch noch weitere Metadaten als Nutzungsinformationen dienen, sodass die Auflistung nicht einschränkend zu verstehen ist.

In einem weiteren Aspekt wird vorgeschlagen, dass zumindest ein Teil der Nutzungsinformationen durch eine Augmented-Reality-Datenbrille erfassbar ist.

Eine Augmented-Reality-Datenbrille ist vorzugsweise eine tragbare, brillenähnliche elektronische Vorrichtung, die digitale Informationen in das Sichtfeld des Nutzers einblendet und mit der realen Umgebung überlagert. Eine Augmented-Reality-Datenbrille ermöglicht vorzugsweise die Darstellung virtueller Elemente, wie Anleitungen, Hinweise oder interaktive Bedienelemente, während der Nutzer seine Umgebung weiterhin wahrnimmt. Eine Augmented-Reality-Datenbrille kann vorzugsweise Sensoren, Kameras und Bewegungserkennungssysteme enthalten, um Inhalte dynamisch an die Blickrichtung und Umgebung des Nutzers anzupassen. Eine Augmented-Reality-Datenbrille wird vorzugsweise zur Unterstützung von Arbeitsprozessen, der Navigation, der Informationsanzeige oder zur Interaktion mit smarten Geräten eingesetzt. Eine Augmented-Reality-Datenbrille kann vorzugsweise mit dem Küchengerät verbunden sein, um Zubereitungsschritte, Rezeptinformationen oder Bedienhinweise direkt im Sichtfeld des Nutzers anzuzeigen. Eine Augmented-Reality-Datenbrille kann vorzugsweise über Sprachsteuerung, Gesten und/oder Augenbewegungen bedient werden, um eine freihändige Interaktion zu ermöglichen. Eine Augmented-Reality-Datenbrille kann vorzugsweise individuell anpassbare Inhalte anzeigen, die auf den Präferenzen und Nutzungsinformationen des Nutzers basieren. Ferner können also die Nutzerinformationen auch um Datenströme aus Nutzer-Assistenzsystemen, wie AR-Datenbrillen, ergänzt werden. Dabei kann beispielsweise eine Kamera einen Lebensmitteleinkauf und/oder einen Aufbewahrungsraum für Lebensmittel und/oder einen Kochprozess erfassen und diese Bilddaten als weitere Nutzerinformationen bereitstellen. Aus diesen Daten werden vorzugsweise Informationen extrahiert, die dem Nutzerprofil hinzugefügt werden können, um dieses immer weiter anzureichern.

In einem weiteren Aspekt wird vorgeschlagen, dass das Anlegen des Nutzerprofils durch einen Softwaremodul-Agenten ein Identifizieren und/oder ein Authentifizieren des Nutzers aufweist.

Das Identifizieren und/oder Authentifizieren des Nutzers umfasst vorzugsweise Verfahren zur eindeutigen Erkennung einer Person, um personalisierte Funktionen oder gesicherte Zugriffe bereitzustellen. Das Identifizieren und/oder Authentifizieren des Nutzers kann vorzugsweise durch biometrische Merkmale wie Gesichtserkennung, Fingerabdruck- oder Iris-Scan erfolgen. Das Identifizieren und/oder Authentifizieren des Nutzers kann vorzugsweise über digitale Zugangsdaten wie Benutzername und Passwort oder über eine Zwei-Faktor-Authentifizierung mit zusätzlichen Sicherheitsmechanismen erfolgen. Das Identifizieren und/oder Authentifizieren des Nutzers kann vorzugsweise durch smarte Geräte, tragbare Sensoren oder mobile Endgeräte unterstützt werden, die eine automatische Erkennung ermöglichen. Das Identifizieren und/oder Authentifizieren des Nutzers kann vorzugsweise für personalisierte Einstellungen, den Zugriff auf gespeicherte Nutzerprofile oder sicherheitsrelevante Funktionen eines Gerätes genutzt werden. Das Identifizieren und/oder Authentifizieren des Nutzers kann vorzugsweise in vernetzten Haushalten zur Steuerung von Küchengeräten eingesetzt werden, um individuelle Präferenzen zu berücksichtigen oder den Zugriff auf bestimmte Funktionen zu beschränken. Das Nutzerprofil kann beispielsweise nach einer Benutzeridentifikation automatisch angelegt werden. Dabei ist eine einmalige Identifikation des Nutzers am Küchengerät, z.B. durch eine Profilauswahl, und/oder durch automatische Identifikation mittels eines Kamerasystems möglich. Auch kann eine Benutzeridentifikation unterstützt durch Bluetooth und/oder Ultra-Weitband- (UWB)-Tagging ermöglicht werden. Mit anderen Worten kann das Identifizieren und/oder das Authentifizieren eine Gesichtserkennung und/oder eine Spracherkennung und/oder eine Proximitätserkennung des Nutzers aufweisen.

In einem weiteren Aspekt wird vorgeschlagen, dass das Rechenmodul und/oder das Bereitstellungsmodul jeweils als Softwaremodule in einer Server- oder Cloud-Service-Umgebung ausgebildet sind, und wobei das Küchengerät mit der Server- oder Cloud-Service-Umgebung kommunikativ verbunden ist.

Das Rechenmodul und/oder das Bereitstellungsmodul sind vorzugsweise als Softwaremodule in einer Server- oder Cloud-Service-Umgebung ausgebildet, um eine zentrale Verarbeitung und Bereitstellung von Daten zu ermöglichen. Das Rechenmodul und/oder das Bereitstellungsmodul führen vorzugsweise rechenintensive Prozesse in einer vernetzten Infrastruktur aus, um die Leistungsfähigkeit und Skalierbarkeit der Datenverarbeitung zu optimieren. Das Rechenmodul und/oder das Bereitstellungsmodul sind vorzugsweise so konzipiert, dass sie unabhängig von lokalen Geräten arbeiten und über eine Internetverbindung zugänglich sind. Das Rechenmodul und/oder das Bereitstellungsmodul ermöglichen vorzugsweise eine sichere Speicherung und Verarbeitung von Nutzungsinformationen sowie die Bereitstellung personalisierter Assistenzfunktionen. Das Rechenmodul und/oder das Bereitstellungsmodul sind vorzugsweise in einer Cloud-Service-Umgebung integriert, um eine flexible, ortsunabhängige Nutzung und Synchronisation mit verschiedenen Endgeräten zu ermöglichen. Das Rechenmodul und/oder das Bereitstellungsmodul können vorzugsweise durch skalierbare Ressourcen dynamisch an wechselnde Anforderungen angepasst werden, um eine effiziente Datenverarbeitung und schnelle Bereitstellung von Informationen sicherzustellen.

Die Erfindung umfasst auch ein Computerprogrammprodukt, umfassend Befehle, die bei der Ausführung des Verfahrens durch einen Computer bewirken, dass der Computer das Verfahren ausführt.

Die Erfindung umfasst daneben ein computerlesbares Medium, auf dem das Computerprogrammprodukt gespeichert ist.

Das Küchengerät ist vorzugsweise ein Backofen oder ein Herd oder eine Mikrowelle oder eine Heißluftfritteuse oder ein Dampfgarer oder ein Multikocher oder ein Sous-vide-Gerät oder ein Reiskocher oder ein Induktionskochfeld oder eine Kaffeemaschine oder ein Wasserkocher oder eine Küchenmaschine oder eine Eismaschine.

Ein Ausführungsbeispiel der Erfindung ist in den Zeichnungen rein schematisch dargestellt und wird nachfolgend näher beschrieben. Es zeigt
- Figur 1: zeigt eine schematische Darstellung einer Ausführungsform eines vorliegenden Systems; und
- Figur 2: zeigt ein schematisches Flussdiagramm einer Ausführungsform eines vorliegenden Verfahrens.

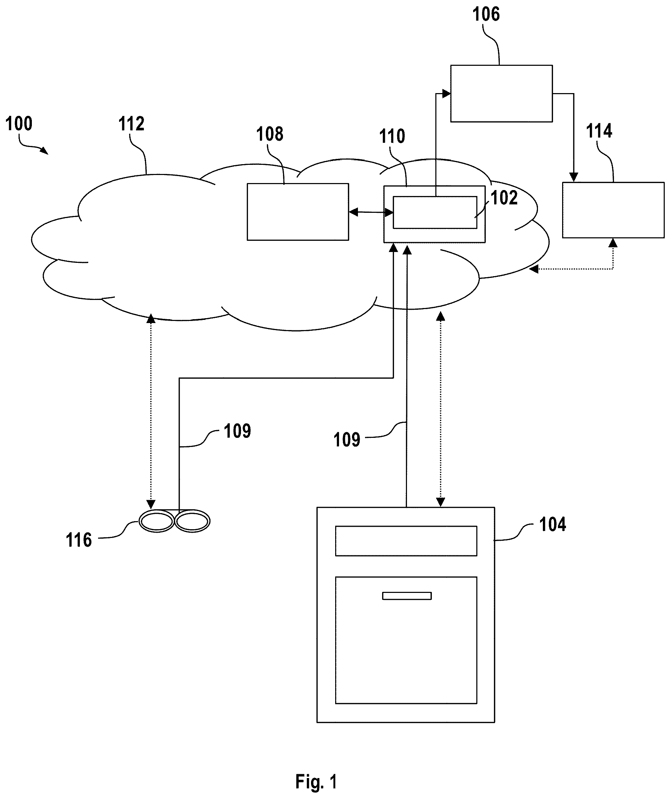

Fig. 1 zeigt eine schematische Darstellung einer Ausführungsform eines vorliegenden Systems 100. Das System 100 ist zum automatischen Anlegen eines Nutzerprofils 102 zur Nutzung eines Küchengerätes 104 und zum Bereitstellen einer Assistenzfunktion 106 ausgelegt.

Hierzu umfasst das System 100 das Küchengerät 104, ein Rechenmodul 108 zur Ausführung eines Softwaremodul-Agenten und ein Bereitstellungsmodul 110. Das Küchengerät 104 ist vorliegend ein Backofen. Das Küchengerät 104 kann aber auch ein Herd oder eine Mikrowelle oder eine Heißluftfritteuse oder ein Dampfgarer oder ein Multikocher oder ein Sous-vide-Gerät oder ein Reiskocher oder ein Induktionskochfeld oder eine Kaffeemaschine oder ein Wasserkocher oder eine Küchenmaschine oder eine Eismaschine sein.

Das Bereitstellungsmodul 110 ist dazu ausgebildet, Nutzungsinformationen 109 zur Nutzung des Küchengerätes 104 und/oder zu einem Nutzer des Küchengerätes 104 und/oder Lebensmitteln, die mit dem Küchengerät 104 zubereitet werden, bereitzustellen. Das Rechenmodul 108 ist dazu ausgebildet, das Nutzerprofil 102 mittels des Softwaremodul-Agenten unter Verwendung der bereitgestellten Nutzerinformationen anzulegen, wobei das Bereitstellungsmodul 110 dazu ausgebildet ist, die Assistenzfunktion 106 unter Verwendung des angelegten Nutzerprofils 102 bereitzustellen.

Die Nutzungsinformationen 109 können beispielsweise Informationen zu Lebensmitteln und/oder zu zubereiteten Lebensmitteln und/oder zu einem Rezept und/oder zu einem Rezeptergebnis und/oder zu einem an dem Küchengerät 104 ausgewählten Automatikprogramm zum Zubereiten der Lebensmittel und/oder zu einer Geräteeinstellung des Küchengerätes 104 und/oder zu Bild- und/oder Textdaten von den Lebensmitteln und/oder dem Rezept und/oder zu gelagerten Lebensmitteln und/oder zu Lebensmitteleinkäufen und/oder zu manuellen Nutzerinformationen aufweisen.

Das Rechenmodul 108 und das Bereitstellungsmodul 110 sind jeweils als Softwaremodule in einer Server- oder Cloud-Service-Umgebung 112 ausgebildet. Das Küchengerät 104 ist dabei ebenfalls mit der Server- oder Cloud-Service-Umgebung 112 kommunikativ verbunden (angezeigt durch Doppelpfeil).

Die bereitgestellte Assistenzfunktion 106 assistiert unter Verwendung des angelegten Nutzerprofils 102 dem Nutzer bei einer Auswahl von Lebensmitteln für die Zubereitung eines Gerichtes und/oder bei einem Einkauf von Lebensmitteln und/oder bei einer Auswahl eines mit dem Küchengerät 104 zuzubereitenden Rezeptes und/oder bei einer Anpassung eines Rezeptes und/oder bei der Durchführung einer Lebensmittelzubereitung und/oder bei der Einhaltung diätischer Bedürfnisse und/oder bei einer Ernährungsberatung.

Ferner kann die Assistenzfunktion 106 unter Verwendung des angelegten Nutzerprofils 102 zumindest Teile der Nutzungsinformationen 109 und/oder des Nutzerprofils 102 über eine öffentliche Plattform 114 mit anderen Nutzern teilt und/oder für andere Nutzer einsehbar macht, um dem Nutzer und/oder den anderen Nutzern der öffentlichen Plattform 114 bei der Auswahl von Lebensmitteln und/oder Rezepten zu assistieren und/oder über die öffentliche Plattform 114 bereitgestellte Rezepte auf Basis des angelegten Nutzerprofils 102 des Nutzers und/oder auf Basis von Nutzerprofilen der anderen Nutzer anzupassen.

Dem Nutzer kann auf Basis von Informationen der öffentlichen Plattform 114, die zumindest teilweise den Teilen der Nutzungsinformationen 109 und/oder des Nutzerprofils 102 entsprechen, ein Anpassungsvorschlag zur Anpassung einer Geräteeinstellung des Küchengerätes 104 ausgegeben oder die Anpassung der Geräteeinstellung automatisch an dem Küchengerät 104 vorgenommen werden. Der Nutzer kann ferner durch eine Nutzerrückmeldung über die öffentliche Plattform 114 eine Bewertung zu einem Automatikprogramm und/oder zu einer Einstellung des Automatikprogramms des Küchengerätes 104 abgeben, auf deren Basis eine Anpassung des Automatikprogramms erfolgt.

Das System kann ferner eine Augmented-Reality-Datenbrille 116 aufweisen, durch die zumindest ein Teil der Nutzungsinformationen 109 erfassbar ist. Die Augmented-Reality-Datenbrille 116 ist ebenfalls mit der Server- oder Cloud-Service-Umgebung 112 kommunikativ verbunden (angezeigt durch Doppelpfeil).

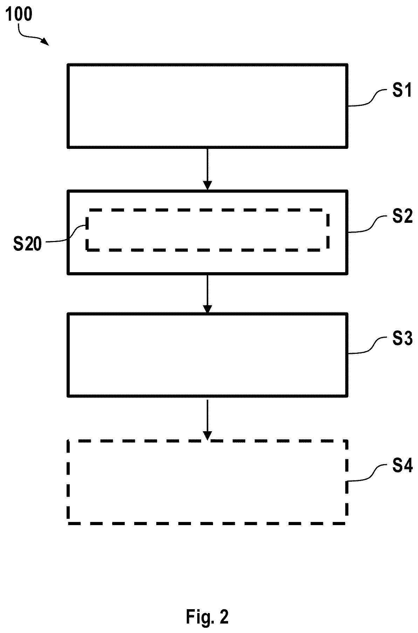

In Fig. 2 ist ein schematisches Flussdiagramm eines Ausführungsbeispiels des Verfahrens gezeigt. In einem Schritt S1 erfolgt ein Bereitstellen von Nutzungsinformationen 109 zur Nutzung des Küchengerätes 104 und/oder zu einem Nutzer des Küchengerätes 104 und/oder Lebensmitteln, die mit dem Küchengerät 104 zubereitet werden. In einem Schritt S2 erfolgt ein Anlegen des Nutzerprofils 102 durch einen Softwaremodul-Agenten unter Verwendung der bereitgestellten Nutzerinformationen. In einem Schritt S3 erfolgt ein Bereitstellen der Assistenzfunktion unter Verwendung des angelegten Nutzerprofils 102.

Das Anlegen S2 des Nutzerprofils 102 durch einen Softwaremodul-Agenten kann optional ein Identifizieren und/oder ein Authentifizieren S20 des Nutzers aufweisen. Das optionale Identifizieren und/oder das Authentifizieren S20 kann wiederum eine Gesichtserkennung und/oder eine Spracherkennung und/oder eine Proximitätserkennung des Nutzers aufweisen.

Ferner kann das Verfahren optional ein Erkennen S4 von Rezepten in einer Mehrzahl von Nutzerprofilen, die durch eine vorbestimmte Anzahl von Nutzern der öffentlichen Plattform 114 durchgeführt werden, aufweisen. Die erkannten Rezepte werden durch ein, insbesondere generatives, maschinelles Lernmodell analysiert, um Parameter und/oder Informationen aus den Rezepten zu extrahieren. Durch den Softwaremodul-Agenten wird aus den erkannten oder analysierten Rezepten ein Automatikprogramm für das Küchengerät 104 erstellt, das durch das Küchengerät 104 ausführbar ist.

### Bezugszeichenliste

- 100: System
- 102: Nutzerprofil
- 104: Küchengerätes
- 106: Assistenzfunktion
- 108: Rechenmodul
- 109: Nutzungsinformationen
- 110: Bereitstellungsmodul
- 112: Server- oder Cloud-Service-Umgebung
- 114: öffentliche Plattform
- 116: Augmented-Reality-Datenbrille

- S1: Verfahrensschritt
- S2: Verfahrensschritt
- S20: Verfahrensschritt
- S3: Verfahrensschritt
- S4: Verfahrensschritt

## Patentansprüche

1. Verfahren zum automatischen Anlegen eines Nutzerprofils (102) zur Nutzung eines Küchengerätes (104) und zum Bereitstellen einer Assistenzfunktion (106), das Verfahren aufweisend die Schritte:
- Bereitstellen (S1) von Nutzungsinformationen (109) zur Nutzung des Küchengerätes (104) und/oder zu einem Nutzer des Küchengerätes (104) und/oder Lebensmitteln, die mit dem Küchengerät (104) zubereitet werden;
- Anlegen (S2) des Nutzerprofils durch einen Softwaremodul-Agenten unter Verwendung der bereitgestellten Nutzerinformationen; und
- Bereitstellen (S3) der Assistenzfunktion (106) unter Verwendung des angelegten Nutzerprofils (102).

2. Verfahren nach Anspruch 1, wobei die bereitgestellte Assistenzfunktion (106) unter Verwendung des angelegten Nutzerprofils (102) dem Nutzer bei einer Auswahl von Lebensmitteln für die Zubereitung eines Gerichtes und/oder bei einem Einkauf von Lebensmitteln und/oder bei einer Auswahl eines mit dem Küchengerät zuzubereitenden Rezeptes und/oder bei einer Anpassung eines Rezeptes und/oder bei der Durchführung einer Lebensmittelzubereitung und/oder bei der Einhaltung diätischer Bedürfnisse und/oder bei einer Ernährungsberatung assistiert.

3. Verfahren nach Anspruch 1 oder 2, wobei die bereitgestellte Assistenzfunktion (106) unter Verwendung des angelegten Nutzerprofils (102) zumindest Teile der Nutzungsinformationen (109) und/oder des Nutzerprofils (102) über eine öffentliche Plattform (114) mit anderen Nutzern teilt und/oder für andere Nutzer einsehbar macht, um dem Nutzer und/oder den anderen Nutzern der öffentlichen Plattform (114) bei der Auswahl von Lebensmitteln und/oder Rezepten zu assistieren und/oder über die öffentliche Plattform (114) bereitgestellte Rezepte auf Basis des angelegten Nutzerprofils (102) des Nutzers und/oder auf Basis von Nutzerprofilen der anderen Nutzer anzupassen.

4. Verfahren nach Anspruch 3, wobei ferner ein Erkennen (S4) von Rezepten in einer Mehrzahl von Nutzerprofilen, die durch eine vorbestimmte Anzahl von Nutzern der öffentlichen Plattform (114) durchgeführt werden, erfolgt.

5. Verfahren nach Anspruch 4, wobei die erkannten Rezepte durch ein, insbesondere generatives, maschinelles Lernmodell analysiert werden, um Parameter und/oder Informationen aus den Rezepten zu extrahieren.

6. Verfahren nach Anspruch 4 oder 5, wobei durch den Softwaremodul-Agenten aus den erkannten oder analysierten Rezepten ein Automatikprogramm für das Küchengerät (104) erstellt wird, das durch das Küchengerät (104) ausführbar ist.

7. Verfahren nach einem der Ansprüche 3 bis 6, wobei dem Nutzer auf Basis von Informationen der öffentlichen Plattform (114), die zumindest teilweise den Teilen der Nutzungsinformationen (109) und/oder des Nutzerprofils (102) entsprechen, ein Anpassungsvorschlag zur Anpassung einer Geräteeinstellung des Küchengerätes (104) ausgegeben oder die Anpassung der Geräteeinstellung automatisch an dem Küchengerät (104) vorgenommen wird.

8. Verfahren nach einem der Ansprüche 3 bis 7, wobei der Nutzer durch eine Nutzerrückmeldung über die öffentliche Plattform (114) eine Bewertung zu einem Automatikprogramm und/oder zu einer Einstellung des Automatikprogramms des Küchengerätes (104) abgibt, auf deren Basis eine Anpassung des Automatikprogramms erfolgt.

9. Verfahren nach einem der vorstehenden Ansprüche, wobei die bereitgestellte Assistenzfunktion (106) unter Verwendung des angelegten Nutzerprofils (102) zur Kommunikation mit einer mobilen Applikation ausgebildet ist, um dem Nutzer über die mobile Applikation mit Hinweisen zu assistieren.

10. Verfahren nach einem der vorstehenden Ansprüche, wobei die Nutzungsinformationen (109) Informationen zu Lebensmitteln und/oder zu zubereiteten Lebensmitteln und/oder zu einem Rezept und/oder zu einem Rezeptergebnis und/oder zu einem an dem Küchengerät (104) ausgewählten Automatikprogramm zum Zubereiten der Lebensmittel und/oder zu einer Geräteeinstellung des Küchengerätes (104) und/oder zu Bild- und/oder Textdaten von den Lebensmitteln und/oder dem Rezept und/oder zu gelagerten Lebensmitteln und/oder zu Lebensmitteleinkäufen und/oder zu manuellen Nutzerinformationen aufweist.

11. Verfahren nach einem der vorstehenden Ansprüche, wobei zumindest ein Teil der Nutzungsinformationen (109) durch eine Augmented-Reality-Datenbrille (116) erfassbar ist.

12. Verfahren nach einem der vorstehenden Ansprüche, wobei das Anlegen (S2) des Nutzerprofils (102) durch einen Softwaremodul-Agenten ein Identifizieren und/oder ein Authentifizieren (S20) des Nutzers aufweist.

13. Verfahren nach Anspruch 12, wobei das Identifizieren und/oder das Authentifizieren (S20) eine Gesichtserkennung und/oder eine Spracherkennung und/oder eine Proximitätserkennung des Nutzers aufweist.

14. System (100) zum automatischen Anlegen eines Nutzerprofils (102) zur Nutzung eines Küchengerätes (104) und zum Bereitstellen einer Assistenzfunktion (106), das System (100) aufweisend das Küchengerät (104), eine Rechenmodul (108) zur Ausführung eines Softwaremodul-Agenten und ein Bereitstellungsmodul (110), wobei das Bereitstellungsmodul (110) dazu ausgebildet ist, Nutzungsinformationen (109) zur Nutzung des Küchengerätes (104) und/oder zu einem Nutzer des Küchengerätes (104) und/oder Lebensmitteln, die mit dem Küchengerät (104) zubereitet werden, bereitzustellen, wobei das Rechenmodul (108) dazu ausgebildet ist, das Nutzerprofil (102) mittels des Softwaremodul-Agenten unter Verwendung der bereitgestellten Nutzerinformationen anzulegen, wobei das Bereitstellungsmodul (110) dazu ausgebildet ist, die Assistenzfunktion (106) unter Verwendung des angelegten Nutzerprofils (102) bereitzustellen.

15. System (100) nach Anspruch 14, wobei das Rechenmodul (108) und/oder das Bereitstellungsmodul (110) jeweils als Softwaremodule in einer Server- oder Cloud-Service-Umgebung (112) ausgebildet sind, und wobei das Küchengerät (104) mit der Server- oder Cloud-Service-Umgebung (112) kommunikativ verbunden ist.
